# FASCICULE DE BREVET EUROPEEN

(11) **EP 1 079 877 B1**
(45) Date de publication et mention de la délivrance du brevet: **07.12.2005**
(21) Numéro de dépôt: 99919348.5
(22) Date de dépôt: 18.05.1999
(51) Int. Cl.: A61M 5/24, A61M 5/32

(54) **SERINGUE D'INJECTION A PROTECTEUR D'AIGUILLE CHARGE PAR UN RESSORT**
INJEKTIONSSPRITZE MIT EINEM FEDERBEAUFSCHLAGTEN NADELSCHUTZ
INJECTION SYRINGE WITH NEEDLE SHIELD LOADED WITH A SPRING

(30) Priorité: 19.05.1998 FR 9806321
(43) Date de publication de la demande: 07.03.2001
(73) Titulaire: SEDAT, 69540 Irigny (Rhône) (FR)
(72) Inventeur: PEROUSE, Eric, F-95290 L'Isle Adam (FR); ARNISSOLLE, Yves, F-69230 Saint Genis Laval (FR)
(74) Mandataire: Blot, Philippe Robert Emile
(86) Numéro de dépôt international: PCT/FR1999/001182
(87) Numéro de publication internationale: WO 1999/059658

(56) Documents cités:
- WO-A-97/14455
- FR-A- 2 741 268
- GB-A- 728 248
- US-A- 4 795 432

## Description

La présente invention concerne une seringue d'injection du type comportant un corps de seringue portant une aiguille d'injection, un réservoir contenant un liquide à injecter, lequel réservoir comporte une partie mobile montée déplaçable à coulissement par rapport au corps pour provoquer la circulation du liquide à injecter au travers de l'aiguille d'injection et un protecteur d'aiguille mobile monté déplaçable par rapport à l'aiguille d'injection sous la commande d'un ressort, depuis une position escamotée en retrait de l'extrémité d'injection de l'aiguille, vers une position active de protection, dans laquelle l'extrémité avant du protecteur se trouve en avant de l'extrémité d'injection de l'aiguille, la seringue comprenant en outre des moyens de retenue pendant l'injection d'une première extrémité du ressort coopérant avec le protecteur, et des moyens portés par ladite partie mobile, de libération en fin d'injection de ladite première extrémité du ressort pour le déplacement du protecteur jusqu'à sa position active de protection.

On connaît, par exemple du document EP-A-0.467.173, une seringue du type précité. Dans une telle seringue, le protecteur d'aiguille est disposé autour du corps de seringue dont il constitue un fourreau. Le ressort de commande du protecteur d'aiguille est initialement comprimé entre le corps de seringue et le fourreau. Le fourreau est retenu par enclenchement élastique sur le corps de seringue. Le piston de seringue comporte des moyens de désolidarisation du fourreau par rapport au corps de seringue, ce qui assure la libération du ressort et le déplacement du fourreau jusqu'à sa position active de protection.

Le ressort étant initialement comprimé, celui-ci doit être comprimé lors de la fabrication de la seringue, ce qui rend son montage délicat. De plus, le ressort étant maintenu comprimé lors du stockage de la seringue, celui-ci peut être l'objet d'un vieillissement prématuré, réduisant ses performances avec le temps.

Les caractéristiques du préambule de la revendication 1 sont connues du document WO-A-97/14455.

L'invention a pour but de proposer une seringue d'injection munie d'un protecteur d'aiguille sollicité par un ressort ne présentant pas les inconvénients mentionnés ci-dessus, et qui, en particulier, soit facile à réaliser et fiable.

A cet effet, l'invention a pour objet une seringue d'injection du type précité, caractérisée en ce que la seconde extrémité du ressort est appliquée contre ladite partie du réservoir mobile par rapport au corps pour la compression progressive du ressort pendant l'injection.

Suivant des modes particuliers de réalisation, la seringue d'injection comporte l'une ou plusieurs des caractéristiques suivantes :
- ladite partie mobile comporte une paroi cylindrique obturée par un fond formant réservoir, dans lequel est monté coulissant un piston traversé par ladite aiguille d'injection, le corps comportant des moyens d'immobilisation du piston au moins lors de l'injection ;
- l'aiguille d'injection s'étend sur l'essentiel de la longueur du corps ;
- ledit ressort est interposé entre ladite partie mobile et le protecteur, et lesdits moyens de retenue de la première extrémité du ressort comportent des moyens d'immobilisation du protecteur par rapport au corps, lesdits moyens de libération de la première extrémité du ressort comportant des moyens de libération du protecteur sur lequel s'applique ladite première extrémité du ressort ;
- le protecteur comporte une bague d'extrémité prolongée par des jambages s'étendant partiellement à l'intérieur du corps, lesdits moyens d'immobilisation du protecteur comportent des profils complémentaires de blocage en saillie et en creux portés par le corps et les jambages, et lesdits moyens de libération du protecteur comportent des moyens de déformation des jambages en vue du dégagement des profils complémentaires en saillie et en creux ; et
- les moyens de déformation comportent, portées par les jambages, des surfaces de came, adaptées pour coopérer avec l'extrémité de ladite partie mobile.

L'invention sera mieux comprise à la lecture de la description qui va suivre, donnée uniquement à titre d'exemple, et faite en se référant aux dessins, sur lesquels :
- La figure 1 est une vue en coupe longitudinale d'une seringue selon l'invention en position de stockage ;
- La figure 2 est une vue en perspective du protecteur d'aiguille de la seringue de la figure 1 ;
- La figure 3 est une vue en coupe longitudinale de la seringue en phase initiale d'injection ;
- La figure 4 est une vue en coupe longitudinale de la seringue pendant l'injection ; et
- La figure 5 est une vue en coupe longitudinale de la seringue, immédiatement après mise en place automatique du protecteur d'aiguille.

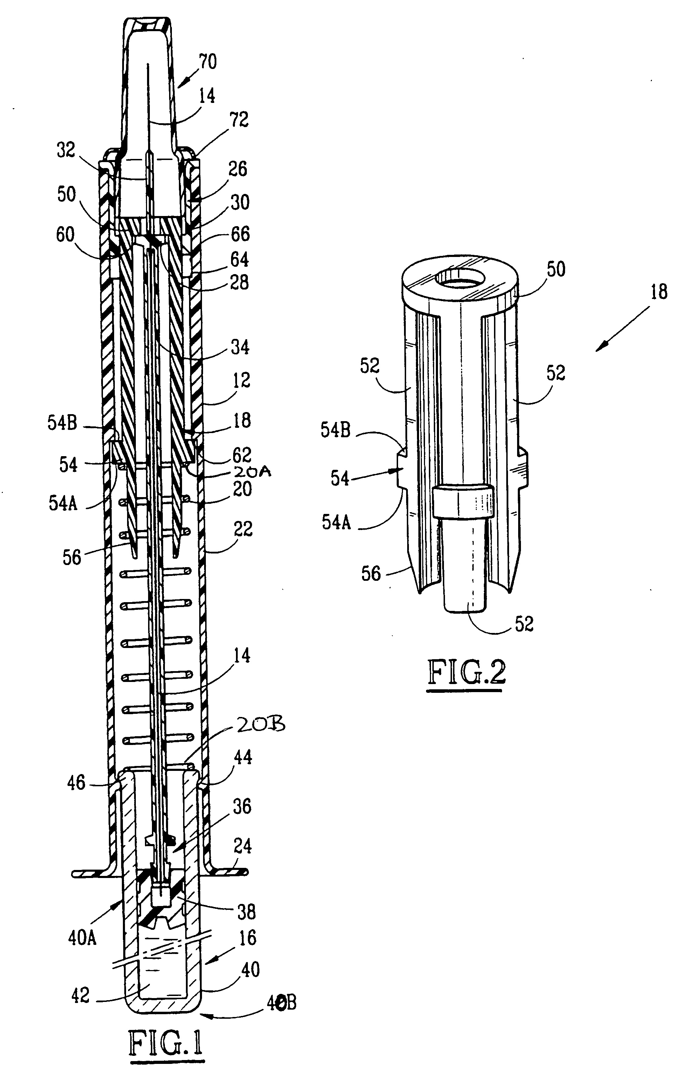

La seringue représentée sur la figure 1 est essentiellement de révolution. Elle comporte un corps de seringue externe 12 portant une aiguille d'injection 14, dont une extrémité d'injection fait saillie à l'avant en dehors du corps 12 et dont l'autre extrémité est destinée à être reçue à l'intérieur d'un réservoir 16 rempli d'un liquide à injecter.

En outre, la seringue comporte un protecteur d'aiguille 18 déplaçable entre une position escamotée, et une position active de protection sous la commande d'un ressort à boudin 20 interposé entre le protecteur d'aiguille 18 et une partie mobile du réservoir 16.

Le corps 12 est constitué d'un tube cylindrique 22 ouvert à ses deux extrémités. Il comporte à l'arrière deux pattes 24 d'application des doigts.

A l'extrémité avant du corps est emmanché un porte-aiguille 26. Celui-ci présente une paroi transversale 28 portant l'aiguille 14. Cette paroi transversale porte extérieurement un collet 30 de solidarisation contre la paroi interne du tube 22. Suivant l'axe du collet 30, l'aiguille 14 est reçue à l'intérieur d'un plot axial 32 auquel elle est collée.

L'aiguille 14 s'étend suivant toute la longueur du corps 12. Dans sa partie reçue à l'intérieur du tube 22, l'aiguille 14 est entourée sur l'essentiel de sa longueur par un fourreau 34 venu de matière avec la paroi transversale 28. L'extrémité arrière de l'aiguille fait saillie axialement au-delà du fourreau 34. Ce dernier comporte à son extrémité des moyens 36 d'enclenchement pour un piston perforable 38 du réservoir 16.

Le réservoir 16 comporte un tube borgne 40 formant une partie mobile de celui-ci. Le tube borgne 40 comporte une paroi latérale cylindrique 40A obturée à une extrémité par un fond 408. Le liquide à injecter, noté 42, est reçu dans le réservoir ainsi formé. Ce réservoir est obturé par le piston perforable 38. Le piston 38 est monté coulissant suivant la longueur du tube borgne 40.

L'extrémité ouverte du tube 40 est engagée, comme représenté sur la figure 1, dans le corps 12 à son extrémité arrière. Ainsi, le piston 38 est reçu en regard de l'extrémité arrière de l'aiguille 14.

Sur sa surface cylindrique interne, le tube 22 comporte des saillies 44 adaptées pour coopérer avec un bourrelet périphérique externe 46 ménagé à l'extrémité ouverte de la paroi cylindrique 40A. Les saillies 44 et le bourrelet 46 assurent le maintien du réservoir 16 dans le corps 12.

Le protecteur d'aiguille 18 est représenté à plus grande échelle sur la figure 2. Il comporte à son extrémité avant une bague rigide 50. Celle-ci est solidaire axialement de trois jambages élastiques 52, chacun défini dans une surface cylindrique prolongeant la bague 50.

Chaque jambage 52 comporte extérieurement une saillie 54 périphérique définissant axialement de part et d'autre deux fronts transversaux 54A, 54B. Les extrémités libres des surfaces latérales externes des jambages 52 définissent des rampes 56 inclinées vers l'axe du protecteur. Ainsi, le protecteur 18 présente à son extrémité un diamètre externe progressivement décroissant vers l'extrémité. Les rampes 56 sont des portions tronconiques.

Comme représenté sur la figure 1, l'essentiel de la longueur des jambages 52 est engagé à l'intérieur du corps 12 au travers d'ouvertures arquées 60 ménagées dans la paroi transversale 28. Seule la bague d'extrémité 50 est maintenue à l'extérieur du corps 12 et entoure le plot 32 supportant l'aiguille.

Sur sa surface cylindrique interne, le tube 22 présente dans sa partie médiane un épaulement 62 orienté vers l'arrière définissant un profil en creux. Celui-ci est adapté pour coopérer avec les fronts 54A définis par les saillies 54, afin d'assurer une retenue, par enclenchement élastique, du protecteur 18 par rapport au corps 12.

De manière analogue, la surface cylindrique interne du tube 22 comporte dans sa partie avant un épaulement 64 orienté vers l'avant. Cet épaulement 64 définit avec le collet 30 une gorge périphérique 66 sur la paroi interne du corps. Cette gorge 66 est adaptée pour recevoir les saillies 54 du protecteur, afin d'assurer son immobilisation dans sa position active de protection, les fronts 54B coopérant alors avec l'épaulement 64.

Le ressort 20 est interposé, comme représenté sur la figure 1, entre l'extrémité de la partie mobile 40 du réservoir et le protecteur d'aiguille 18. Plus précisément, une première extrémité du ressort 20A s'appuie sur les fronts arrière 54B des saillies 54 et la seconde extrémité du ressort 20B s'appuie sur l'extrémité du tube borgne 40 suivant l'épaisseur de celui-ci.

Enfin, la seringue comporte à son extrémité avant un capuchon 70 de protection de l'extrémité d'injection de l'aiguille 14. Ce capuchon est maintenu par des griffes élastiques 72 venues de matière avec le porte-aiguille 26.

La seringue selon l'invention fonctionne de la manière suivante.

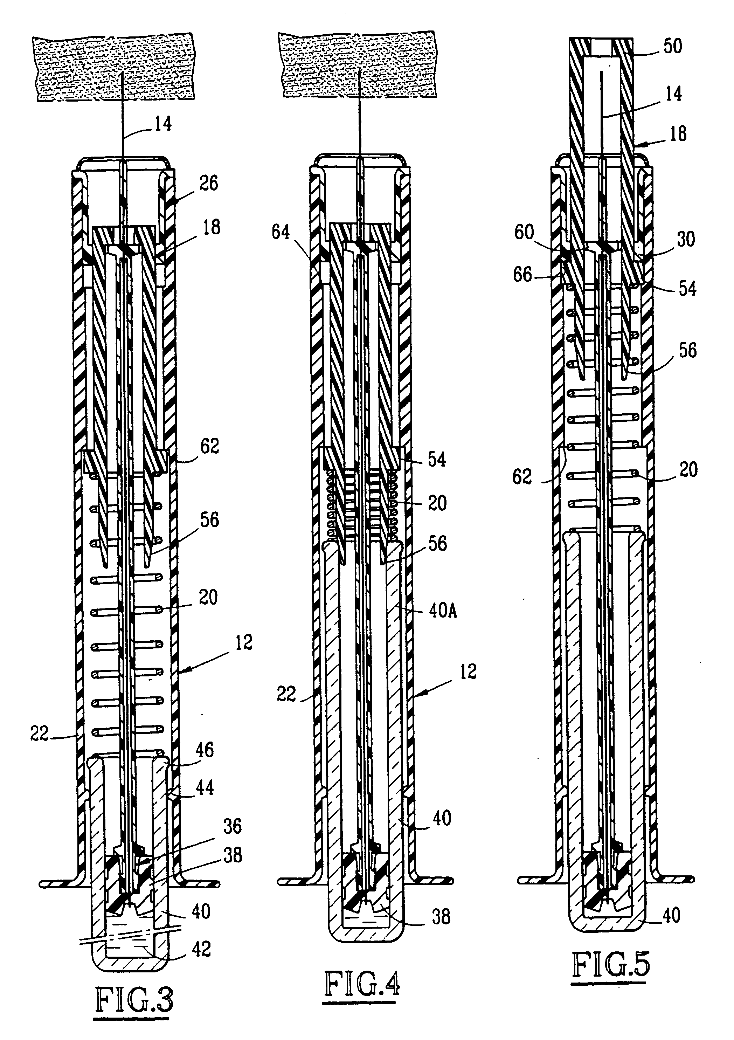

Afin de procéder à une injection, le praticien retire par traction axiale le capuchon 70. Maintenant la seringue entre deux doigts appliqués sur les pattes 24, et appliquant le pouce sur le fond 40B du réservoir, il enfonce la partie mobile 40 de celui-ci dans le corps 12, jusqu'à ce que le piston 38 s'empale sur l'extrémité arrière de l'aiguille 14, comme représenté sur la figure 3.

Dans cette position, le liquide 42 contenu dans le réservoir est susceptible d'être évacué au travers de l'aiguille 14. L'extrémité d'injection de l'aiguille 14 est alors piquée dans les chairs du patient.

Pour procéder à l'injection proprement dite, le praticien enfonce progressivement la partie mobile 40 du réservoir à l'intérieur du corps 12. Ainsi, le piston perforé 38 étant maintenu immobilisé à l'extrémité du fourreau 34, le liquide 42 s'écoule progressivement au travers de l'aiguille 14.

Simultanément, lors du déplacement en translation de la partie mobile 40, le ressort 20 est progressivement comprimé jusqu'à un état représenté sur la figure 4 où les spires du ressort sont sensiblement jointives.

Dans cette position, les rampes 56 portées à l'extrémité des jambages 52 sont engagées à l'intérieur de la paroi cylindrique 40A de la partie mobile du réservoir. Ces rampes, qui constituent des surfaces de came, coopérent avec l'extrémité de la partie mobile 40, pour provoquer la déformation des jambages vers l'axe de la seringue. Ainsi, peu avant l'achèvement de l'injection, les saillies 54 sont pratiquement dégagées de l'épaulement périphérique 62.

Lorsque le praticien enfonce encore davantage la partie mobile 40 à l'intérieur du corps 12, les saillies 54 retenant initialement le protecteur d'aiguille 18 sont totalement dégagées de l'épaulement 62. Ainsi, le protecteur d'aiguille 18 est projeté vers l'avant sous l'action du ressort comprimé 20, les moyens le retenant jusqu'alors étant libérés.

Le protecteur d'aiguille 18 se déplace alors, sous l'action du ressort 20 se détendant progressivement, jusqu'à sa position d'obturation représentée sur la figure 5. Dans celle-ci, son extrémité formée par la bague 50 se situe au-delà de l'extrémité d'injection de l'aiguille 14. Dans cette position, les saillies 54 sont reçues élastiquement dans la gorge périphérique 66 portée par le corps. Ainsi, le protecteur d'aiguille 18 est maintenu par enclenchement élastique dans sa position active de protection.

Cet enclenchement élastique du protecteur évite tout risque de piqûre accidentelle depuis l'extrémité d'injection de l'aiguille.

On conçoit qu'avec une telle seringue, le ressort d'actionnement du protecteur d'aiguille n'est comprimé que pendant un bref laps de temps, le ressort étant progressivement comprimé lors de l'injection et immédiatement libéré en fin d'injection.

Lors des phases de stockage de la seringue, le ressort n'est que peu comprimé, celui-ci n'ayant pas à emmagasiner l'énergie nécessaire au déplacement ultérieur du protecteur d'aiguille. Ainsi, les risques de vieillissement prématuré du ressort et la difficulté de montage de la seringue sont évités.

## Revendications

1. Seringue d'injection, comportant un corps (12) de seringue portant une aiguille d'injection (14), un réservoir (16) contenant un liquide (42) à injecter, lequel réservoir (16) comporte une partie mobile (40) montée déplaçable à coulissement par rapport au corps (12) pour provoquer la circulation du liquide à injecter au travers de l'aiguille d'injection (14), un protecteur d'aiguille mobile (18) monté déplaçable par rapport à l'aiguille d'injection (14) et un ressort (20) de commande du protecteur d'aiguille (18) pour son déplacement sous la commande du ressort (20), depuis une position escamotée en retrait de l'extrémité d'injection de l'aiguille (14), vers une position active de protection, dans laquelle l'extrémité avant du protecteur (18) se trouve en avant de l'extrémité d'injection de l'aiguille (14), la seringue comprenant en outre des moyens (54, 62) de retenue, pendant l'injection, d'une première extrémité (20A) du ressort (20) coopérant avec le protecteur, et des moyens portés par ladite partie mobile (40), de libération en fin d'injection de ladite première extrémité (20A) du ressort (20) pour le déplacement du protecteur (18) jusqu'à sa position active de protection, **caractérisée en ce que** la seconde extrémité (20B) du ressort (20) est appliquée contre ladite partie du réservoir (40) mobile par rapport au corps (12) pour la compression progressive du ressort (20) pendant l'injection.

2. Seringue d'injection selon la revendication 1, **caractérisée en ce que** ladite partie mobile (40) comporte une paroi cylindrique (40A) obturée par un fond (40B) formant réservoir, dans lequel est monté coulissant un piston (38) traversé par ladite aiguille d'injection (14), le corps comportant des moyens (36) d'immobilisation du piston au moins lors de l'injection.

3. Seringue d'injection selon la revendication 2, **caractérisée en ce que** l'aiguille d'injection (14) s'étend sur l'essentiel de la longueur du corps (12).

4. Seringue d'injection selon l'une quelconque des revendications précédentes, **caractérisée en ce que** ledit ressort (20) est interposé entre ladite partie mobile (40) et le protecteur (18), et **en ce que** lesdits moyens de retenue de la première extrémité du ressort comportent des moyens d'immobilisation (54, 62) du protecteur (18) par rapport au corps (12), lesdits moyens de libération de la première extrémité (20A) du ressort (20) comportant des moyens (56) de libération du protecteur (81) sur lequel s'applique ladite première extrémité (20A) du ressort (20).

5. Seringue d'injection selon la revendication 4, **caractérisée en ce que** le protecteur (18) comporte une bague d'extrémité (50) prolongée par des jambages (52) s'étendant partiellement à l'intérieur du corps (12), **en ce que** lesdits moyens d'immobilisation du protecteur (18) comportent des profils complémentaires de blocage en saillie et en creux (54, 62) portés par le corps (12) et les jambages (52), et **en ce que** lesdits moyens de libération du protecteur comportent des moyens (56) de déformation des jambages (52) en vue du dégagement des profils complémentaires en saillie et en creux (54, 62).

6. Seringue d'injection selon la revendication 5, **caractérisée en ce que** les moyens de déformation comportent, portées par les jambages (52), des surfaces de came (56), adaptées pour coopérer avec l'extrémité de ladite partie mobile (40).

## Patentansprüche

1. Injektionsspritze, umfassend einen eine Injektionsnadel (14) tragenden Spritzenkörper (12), einen eine zu injizierende Flüssigkeit (42) enthaltenden Behälter (16), der einen beweglichen Teil (40) besitzt, der bezüglich des Körpers (12) verschiebbar angeordnet ist, um den Umlauf der zu injizierenden Flüssigkeit durch die Injektionsnadel (14) zu bewirken, einen beweglichen Nadelschutz (18), der bezüglich der Injektionsnadel (14) beweglich angeordnet ist, und eine Feder (20) zur Steuerung des Nadelschutzes (18) für seine Bewegung unter der Beaufschlagung durch die Feder (20) von einer eingefahren, bezüglich des Injektionsendes der Nadel (14) zurückversetzten Stellung in eine aktive Schutzstellung, in der das vordere Ende des Schutzes (18) sich vor dem Injektionsende der Nadel (14) befindet, wobei die Spritze außerdem Mittel (54, 62) zum Zurückhalten eines ersten Endes (20A) der mit dem Schutz zusammenwirkenden Feder (20) während der Injektion und von diesem beweglichen Teil (40) getragene Mittel zum Freigeben des ersten Endes (20A) der Feder (20) am Ende der Injektion für die Bewegung des Schutzes (18) bis in seine aktive Schutzstellung umfasst, **dadurch gekennzeichnet, dass** das zweite Ende (20B) der Feder (20) für die progressive Komprimierung der Feder (20) während der Injektion an den bezüglich des Körpers (12) beweglichen Teil des Behälters (40) angelegt ist.

2. Injektionsspritze nach Anspruch 1, **dadurch gekennzeichnet, dass** der bewegliche Teil (40) eine zylindrische Wand (40A) aufweist, die durch einen einen Behälter bildenden Boden (40B) verschlossen ist, in dem ein von der Injektionsnadel (14) durchsetzter Kolben (38) verschiebbar angeordnet ist, wobei der Körper Mittel (36) zur Blockierung des Kolbens mindestens bei der Injektion umfasst.

3. Injektionsspritze nach Anspruch 2, **dadurch gekennzeichnet, dass** die Injektionsnadel (14) sich über den wesentliche Teil der Länge des Körpers (12) erstreckt.

4. Injektionsspritze nach einem der vorhergehenden Ansprüche, **dadurch gekennzeichnet, dass** die Feder (20) zwischen den beweglichen Teil (40) und den Schutz (18) eingesetzt ist und dass die Mittel zum Zurückhalten des ersten Endes der Feder Mittel (54, 62) zum Blockieren des Schutzes (18) bezüglich des Körpers (12) umfassen, wobei die Mittel zum Freigeben des ersten Endes (20A) der Feder (20) Mittel (56) zum Freigeben des Schutzes (81) aufweisen, an dem das erste Ende (20A) der Feder (20) anliegt.

5. Injektionsspritze nach Anspruch 4, **dadurch gekennzeichnet, dass** der Schutz (18) einen Endring (50) aufweist, der durch Schenkel (52) verlängert ist, die sich teilweise im Inneren des Körpers (12) erstrecken, dass die Mittel zum Blockieren des Schutzes (18) einander ergänzende vorstehende und vertiefte Profile (54, 62) aufweisen, die von dem Körper (12) und den Schenkeln (52) getragen sind, und dass die Mittel zum Freigeben des Schutzes Mittel (56) zum Verformen der Schenkel (52) zum Zweck der Freisetzung der einander ergänzenden vorspringenden und vertieften Profile (54, 62) aufweisen.

6. Injektionsspritze nach Anspruch 5, **dadurch gekennzeichnet, dass** die Verformungsmittel von den Schenkeln (52) getragene Steuerflächen (56) aufweisen, die dafür ausgelegt sind, mit dem Ende des beweglichen Teils (40) zusammenzuwirken.

## Claims

1. Injection syringe comprising a syringe body (12) which supporrs an injection needle (14), a reservoir (16) containing a liquid (42) to be injected, which reservoir (16) comprises a mobile part (40) which is fitted such as to be displaceable in a sliding manner relative to the body (12) in order to give rise to the circulation of the liquid to be injected through the injection needle (14), a mobile needle protector (18) which is fitted such as to be displaceable relative to the injection needle (14), and a spring (20) to control the needle protector (18) for displacement of the latter under the control of the spring (20), from a retracted position in which it is withdrawn from the injection end of the needle (14), to an active protection position in which the front end of the protector (18) is to the front of the injection end of the needle (14), the syringe additionally comprising means (54, 62) for retention, during the injection, of a first end (20A) of the spring (20) which co-operates with the protector, and means which are supported by the said mobile part (40), for release, upon completion of the injection, of the said first end (20A) of the spring (20) for the displacement of the protector (18) to its active protection position, **characterised in that** the second end (20B) of the spring (20) is applied against the said part of the reservoir (40) which is mobile relative to the body (12), for the progressive compression of the spring (20) during the injection.

2. Injection syringe according to claim 1, **characterised in that** the said mobile part (40) comprises a cylindrical wall (40A) which is closed by a base (40B) which forms a reservoir, in which there is fitted, such that it can slide, a piston (38) through which the said injection needle passes (14), the body comprising means (36) for immobilisation of the piston at least during the injection.

3. Injection syringe according to claim 2, **characterised in that** the injection needle (14) extends substantially along the length of the body (12).

4. Injection syringe according to any one of the preceding claims, **characterised in that** the said spring (20) is interposed between the said mobile part (40) and the protector (18), and **in that** the said means for retention or the first end of the spring comprise means (54, 62) for immobilisation of the protector (18) relative to the body (12), the said means for release of the first end (20A) of the spring (20) comprising means (56) for release of the protector (81) onto which the said first end (20A) of the spring (20) is applied.

5. Injection syringe according to claim 4, **characterised in that** the protector (18) comprises an end ring (50) which is extended by legs (52) which extend partially inside the body (12), **in that** the said means for immobilisation of the protector (18) comprise projecting and recessed complementary locking profiles (54, 62) which are supported by the body (12) and the legs (52), and **in that** the said means for release of the protector comprise means (56) for deformation of the legs (52) for the purpose of release of the complementary projecting and recessed profiles (54, 62).

6. Injection syringe according to claim 5, **characterised in that** the means for deformation comprise, supported by the legs (52), cam surfaces (56) which are designed to cooperate with the end of the said mobile part (40).
